Europäisches Patentamt

(19) European Patent Office

Office européen des brevets

(11) Publication number

0 158 499
A2

# (12) EUROPEAN PATENT APPLICATION

(21) Application number: 85302277.0

(22) Date of filing: 02.04.85

(51) Int. Cl.⁴: C 07 C 67/08
C 07 C 69/14

(30) Priority 05.04.84 GB 8408801

(43) Date of publication of application
16.10.85 Bulletin 85/42

(84) Designated Contracting States:
BE DE FR GB IT SE

(71) Applicant BP Chemicals Limited
Belgrave House 76 Buckingham Palace Road
London, SW1W 0SU(GB)

(72) Inventor Russell, John
1 The Ridings
Reigate Surrey RH2 0JE(GB)

(72) Inventor: Stevenson, Alistair James
11 Claremont Avenue
Clitheroe Lancashire(GB)

(74) Representative: Harry, John et al,
BP INTERNATIONAL LIMITED Patents Division Chertsey
Road
Sunbury-on-Thames Middlesex TW16 7LN(GB)

(54) Process for making esters.

(57) Carboxylic acid esters, and in particular ethyl acetate, are produced by esterification of an organic carboxylic acid ester with an alcohol in the presence of an alkyl sulphonic acid having a desulphonation temperature in excess of that of p-toluensulphonic acid. Such sulphonic acids, for example methanesulphonic acid, reduce the rate of fouling of commercially operated esterification units.

EP 0 158 499 A2

1

## PROCESS FOR MAKING ESTERS

The present invention relates to the production of an ester and a in particular a volatile ester by the esterification of an organic carboxylic acid by an alcohol in the presence of, as catalyst, a thermally stable sulphonic acid.

The esterification of an organic carboxylic acid by an alcohol is a reaction which is known to be catalysted by strong inorganic acids such as hydrochloric or sulphuric acid. Such reactions have been described in many textbooks of Organic Chemistry an example of which is "Organic Chemistry Vol I" by I.L. Finar published by Longmans (1963) Chapter 10. On a commercial scale however even at relatively low temperatures fouling of the reactor is a major problem. Fouling restricts heat transfer into the reactor causing the reactor to run inefficiently. Eventually fouling becomes so bad that the reactor has to be shut down and cleaned.

At temperatures corresponding to the boiling point of the esterification reaction mixture at atmospheric pressure or thereabouts, fouling can be reduced by replacing the inorganic acid catalyst by p-toluenesulphonic acid.

However, a major fouling problem arises when the reaction is carried out at higher temperatures, for example at temperatures corresponding to the boiling point of the reaction mixture at superatmospheric pressure. Under these conditions, as described for example in our European Patent Application 0 009 886, severe fouling can occur even with, p-toluenesulphonic acid as a catalyst.

It has now been discovered that reactor fouling at higher

1

temperatures can be substantially reduced by using, as catalyst, a sulphonic acid which has a thermal stability superior to that of p-toluenesulphonic acid. Operation at temperatures in excess of the atmospheric pressure boiling point of the reaction mixture leads to faster reaction rates and greater throughput per unit time.

Accordingly, the present invention provides a process for the production of an ester by reacting an organic carboxylic acid with an alcohol at elevated temperature in the presence of an effective amount of catalyst which comprises a sulphonic acid with a desulphonation temperature greater than that of p-toluenesulphonic acid.

It is preferred that the esterification is carried out in the manner disclosed in our European Patent Application 0 009 886 except that the catalyst disclosed therein is replaced by a sulphonic acid of the type described herein.

Any sulphonic acid which has a desulphonation temperature greater than that of p-toluenesulphonic acid may be used as a catalyst. The desulphonation temperature of a sulphonic acid is defined as 'the minimum temperature at which the reaction (desulphonation) occurs at a practical rate at atmospheric pressure' (see 'Sulphonation and Related Reactions' by E.E. Gilbert, Interscience 1965 P429). The desulphonation temperature of p-toluenesulphonic acid is 186°C hence the sulphonic acids used in the present invention must have desulphonation temperatures in excess of this and preferably in excess of 190°C.

A preferred class of sulphonic acids is the alkyl sulphonic acids which may be represented by the forumla $RSO_3H$ wherein R is a $C_1$ to $C_{12}$ substituted or unsubstituted aliphatic hydrocarbyl group and with the added proviso that the alkyl sulphonic acid has a desulphonation temperature in excess of 186°C. A preferred member of this class of sulphonic acids is methanesulphonic acid ($CH_3SO_3H$) which has a desulphonation temperature in excess of 220°C.

The organic carboxylic acids described in this invention are of the general formula RCOOH where R is a substituted or unsubstituted aliphatic, cycloaliphatic, aryl or heterocyclic hydrocarbyl group. Polycarboxylic acids can also be used. Preferred acids are acetic acid, propionic acid and butanoic acids.

Alcohols are of the form $R_1OH$ wherein $R_1$ is a hydrocarbyl or substituted hydrocarbyl group and suitably the hydrocarbyl group is an aliphatic, cycloaliphatic, aryl or heterocyclic group. Polyols may also be used. Suitable examples of alcohols are methanol, ethanol and propanol, the isomeric butanols and the isomeric pentanols.

The esters formed are produced by reaction of the acid and alcohol feeds. Preferred esters are the 'volatile esters' that is those which can be continuously removed from the reactor by distillation whilst the reaction occurs. An example of a preferred volatile ester is ethyl acetate which is prepared from ethanol and acetic acid.

The sulphonic acid catalyst is added to the reaction mixture so as to comprise from 0.1 to 5 per cent by weight of the reactor contents.

The process described herein may be operated at atmospheric pressure but it is preferably operated at superatmospheric pressure between 1 and 8 bars.

The temperature of esterification may suitably be greater than 80°C and, particularly in the case of ethyl acetate production, in the range 125 to 185°C.

The process may be operated continuously or batchwise. A suitable method for carrying out the esterification continuously is described in our European Patent Application 0 009 886.

The reaction mixture may also contain in addition to the catalyst between 0.1 and 1% by weight of a corrosion inhibitor to reduce corrosion of the vessel. A preferred corrosion inhibitor is copper as a salt for example copper acetate.

The invention will now be described by reference to the following Examples.

Example 1

A mixture which had a composition typical of that found at 'steady-state' in an ethyl acetate reactor was prepared with the following composition:

| Acetic Acid | 81.5% w/w |
| Ethyl acetate | 8.0% w/w |
| Ethanol | 0.5% w/w |
| Water | 10.0% w/w |

To aliquots of this mixture were added copper acetate as corrosion inhibitor (0.1 to 0.2% w/w) together with 2.2% w/w methanesulphonic acid catalyst. The aliquot was charged to a 2-litre horizontal glass kettle fitted with a reflux condenser and refluxed gently (110°C) under nitrogen cover at atmospheric pressure using an external band heater. A 9 x 0.75 inch diameter cylindrical 845 Ti stainless steel probe, immersed in the mixture, was then heated by means of an internal cartridge heater to give an internal probe surface temperature of 178°C. The resulting heat transfer caused boiling to occur from the external surface of the probe, and these conditions were maintained over the test period. No fouling was observed even after 60 days of continuous operation.

Comparative Test A

The method of the previous test was used except that 4% w/w p-toluenesulphonic acid (equivalent to the amount of catalysts used in example 1) replaced methanesulphonic acid as catalyst. In this case severe fouling was observed to take place after 18 days on stream.

These results show that fouling is greatly reduced when methanesulphonic acid is used as the esterification catalyst in place of p-toluenesulphonic acid.

Example 2

A plant producing ethyl acetate by esterification was operated at 19 psig and at a temperature corresponding to the boiling point of the mixture under these conditions. Methanesulphonic acid was used as catalyst at a concentration of 2.2% by weight. The plant was operated for 85 days under these conditions during which maximum ester production was maintained with no fouling.

Comparative Test B

The plant operation described in Example 2 was followed except that 5% by weight p-toluenesulphonic acid was used as catalyst. Esterproduction deteriorated after approximately 10 days on stream and after 18 days extreme fouling caused reactor shutdown.

Claims:

1. A process for the production of an ester by reacting an organic carboxylic acid with an alcohol at elevated temperature in the presence of an effective amount of a catalyst which comprises a sulphonic acid with a desulphonation temperature greater than that of p-toluenesulphonic acid.

2. A process as claimed in Claim 1 wherein the sulphonic acid has a desulphonation temperature in excess of 190°C.

3. A process as claimed in Claim 2 wherein the sulphonic acid is a $C_1$ to $C_{12}$ alkyl sulphonic acid.

4. A process as claimed in claim 3 wherein the sulphonic acid is methanesulphonic acid.

5. A process as claimed in claim 1 wherein the organic carboxylic acid is selected from the group consisting of acetic acid, propionic acid and butanoic acids.

6. A process as claimed in claim 1 wherein the alcohol is selected from the group consisting of methanol, ethanol, propanol, the isomeric butanols and the isomeric pentanols.

7. A process for the production of ethyl acetate by reacting acetic acid with ethanol at elevated temperature in the presence of an effective amount of a catalyst which comprises a sulphonic acid with a desulphonation temperature greater than that of p-toluenesulphonic acid.

8. A process as claimed in claim 7 wherein the elevated temperature is in the range 125 to 185°C.

9. A process as claimed in claim 7 wherein the pressure is in the range 1 to 8 bars.

5

10. A process as claimed in claim 7 wherein a corrosion inhibitor is added.

11. A process as claimed in claim 7 wherein the sulphonic acid is methanesulphonic acid.